(19) Europäisches Patentamt

European Patent Office

Office européen des brevets

(11) **EP 1 028 718 B1**

(12) **EUROPEAN PATENT SPECIFICATION**

(45) Date of publication and mention
of the grant of the patent:
**16.04.2003 Bulletin 2003/16**

(51) Int Cl.[7]: **A61K 31/135**, A61K 9/16,
A61K 9/50

(21) Application number: **98956487.7**

(22) Date of filing: **03.11.1998**

(86) International application number:
**PCT/US98/23338**

(87) International publication number:
**WO 99/022724 (14.05.1999 Gazette 1999/19)**

(54) **EXTENDED RELEASE FORMULATION CONTAINING VENLAFAXIN**

VENLAFAXINHALTIGES ARZNEIMITTEL MIT VERLÄNGERTER WIRKSTOFFABGABE

PREPARATION A LIBERATION PROLONGEE

(84) Designated Contracting States:
**AT BE CH CY DE DK ES FI FR GB GR IE IT LI LU
NL PT SE**
Designated Extension States:
**AL LT LV MK RO SI**

(30) Priority: **05.11.1997 US 964328**

(43) Date of publication of application:
**23.08.2000 Bulletin 2000/34**

(73) Proprietor: **Wyeth
Madison, New Jersey 07940-0874 (US)**

(72) Inventors:
• **SHERMAN, Deborah Marie
Plattsburgh, NY 12901 (US)**
• **CLARK, John Clifton
Peru, NY 12972 (US)**
• **LAMER, John Ulrick
St. Albans, VT 05478 (US)**
• **WHITE, Stephen Andrew
New York 12979 (US)**

(74) Representative: **Connelly, Michael John et al
c/o Patent Department
Wyeth Laboratories
Huntercombe Lane South
Taplow
Maidenhead Berkshire SL6 0PH (GB)**

(56) References cited:
EP-A- 0 797 991 WO-A-94/27589
WO-A-97/37640 US-A- 4 138 475

**Description**

Background of the Invention

Extended release drug formulations are conventionally produced as compressed tablets by hydrogel tablet technology. To produce these sustained release tablet drug dosage forms, the active ingredient is conventionally compounded with cellulose ethers such as methyl cellulose, ethyl cellulose or hydroxypropylmethylcellulose with or without other excipients and the resulting mixture is pressed into tablets. When the tablets are orally administered, the cellulose ethers in the tablets swell upon hydration from moisture in the digestive system, thereby limiting exposure of the active ingredient to moisture. As the cellulose ethers are gradually leached away by moisture, water more deeply penetrates the gel matrix and the active ingredient slowly dissolves and diffuses through the gel, making it available for absorption by the body. An example of such a sustained release tablet dosage form of the analgesic/antiinflammatory drug etodolac (Lodine®) appears in US patent 4,966,768. US patent 4,389,393 discloses sustained release therapeutic compressed solid unit dose forms of an active ingredient plus a carrier base comprised of a high molecular weight hydroxypropylmethylcellulose, methyl cellulose, sodium carboxymethylcellulose and or other cellulose ether.

Where the production of tablets is not feasible, it is conventional in the drug industry to prepare encapsulated drug formulations which provide extended or sustained release properties. In this situation, the extended release capsule dosage forms may be formulated by mixing the drug with one or more binding agents to form a uniform mixture which is then moistened with water or a solvent such as ethanol to form an extrudable plastic mass from which small diameter, typically 1 mm, cylinders of drug/matrix are extruded, chopped into appropriate lengths and transformed into spheroids using standard spheronization equipment. The spheroids, after drying, may then be film-coated to retard dissolution. Gelatin capsules are filled with the film-coated spheroids in the quantity needed to obtain the desired therapeutic effect. Spheroids releasing the drug at different rates may be combined in a gelatin capsule to obtain desired release rates and blood levels. US patent 4,138,475 discloses a sustained release pharmaceutical composition consisting of a hard gelatin capsule filled with film-coated spheroids comprised of propanolol in admixture with microcrystalline cellulose wherein the film coating is composed of ethyl cellulose, optionally, with hydroxypropylmethylcellulose and/or a plasticizer.

Venlafaxine, 1-[2-(dimethylamino)- 1-(4-methoxyphenyl)ethyl]cyclohexanol, is an important drug in the neuropharmacological arsenal used for treatment of depression. Venlafaxine and the acid addition salts thereof are disclosed in US patent 4,535,186. Venlafaxine hydrochloride is presently administered to adults in compressed tablet form in doses ranging from 75 to 350 mg/day, in divided doses two or three times a day. In therapeutic dosing with venlafaxine hydrochloride tablets, rapid dissolution results in a rapid increase in blood plasma levels of the active compound shortly after administration followed by a decrease in blood plasma levels over several hours as the active compound is eliminated or metabolized, until sub-therapeutic plasma levels are approached after about twelve hours following administration, thus requiring additional dosing with the drug. With the plural daily dosing regimen, the most common side effect is nausea, experienced by about forty five percent of patients under treatment with venlafaxine hydrochloride. Vomiting also occurs in about seventeen percent of the patients.

EP 0797991 A1 discloses encapsulated venlafaxine sustained release formulations wherein hydroxypropylmethylcellulose was used in making practical venlafaxine-containing spheroids. EP 0797991 A1 states that it was completely unexpected that an extended release formulation containing venlafaxine hydrochloride could be obtained because the hydrochloride of venlafaxine proved to be extremely water soluble. Further, numerous attempts to produce extended release tablets by hydrogel technology proved to be fruitless because the compressed tablets were either physically unstable (poor compressibility or capping problems) or dissolved too rapidly in dissolution studies. Typically, the tablets prepared as hydrogel sustained release formulations gave 40-50% dissolution at 2 hrs, 60-70% dissolution at 4 hrs and 85-100% dissolution at 8 hrs.

EP 0797991 A1 states further that numerous spheroid formulations were prepared using different grades of microcrystalline cellulose and hydroxypropyl methylcellulose, different ratios of venlafaxine hydrochloride and filler, different binders such as polyvinylpyrrolidone, methylcellulose, water, and polyethylene glycol of different molecular weight ranges in order to find a formulation which would provide a suitable granulation mix which could be extruded properly. In the extrusion process, heat buildup occurred which dried out the extrudate so much that it was difficult to convert the extruded cylinders into spheroids. EP 0797991 A1 then states further that addition of hydroxypropylmethylcellulose 2208 to the venlafaxine hydrochloride-microcrystalline cellulose mix made production of spheroids practical.

The invention of this application represents an improvement in the encapsulated, extended release dosage form of venlafaxine disclosed in EP 0797991 A1. Applicants have now discovered that equally suitable venlafaxine-containing sustained release spheroids can be made without the use of hydroxypropylmethylcellulose as disclosed in EP 0797991 A1, particularly for spheroids containing lower amounts of venlafaxine than those specifically disclosed in EP 0797991 A1. The absence of hydroxypropylmethylcellulose in the spheroids enables the manufacturing process for making the spheroids to be improved, particularly for commercial scale production.

Brief Description of the Invention

In accordance with this invention, there are provided spheroid cores comprising venlafaxine hydrochloride and microcrystalline cellulose, said cores being free of hydroxypropylmethylcellulose. The invention further provides extended release (ER), encapsulated formulations containing the referred spheroid cores comprising venlafaxine hydrochloride as the active drug component, which provide in a single dose, a therapeutic blood serum level over a twenty four hour period.

Through administration of the venlafaxine formulation of this invention, there is provided a method for obtaining a flattened drug plasma concentration to time profile, thereby affording a tighter plasma therapeutic range control than can be obtained with multiple daily dosing. In other words, this invention is useful in a method for eliminating the sharp peaks and troughs (hills and valleys) in blood plasma drug levels induced by multiple daily dosing with conventional immediate release venlafaxine hydrochloride tablets. In essence, the plasma levels of venlafaxine hydrochloride rise, after administration of the extended release formulations of this invention, for between about five to about eight hours ( optimally about six hours) and then begin to fall through a protracted, substantially linear decrease from the peak plasma level for the remainder of the twenty four hour period, maintaining at least a threshold therapeutic level of the drug during the entire twenty-four period. In contrast, the conventional immediate release venlafaxine hydrochloride tablets give peak blood plasma levels in 2 to 4 hours. Hence, this invention is useful in a method for moderating the plural blood plasma peaks and valleys attending the pharmacokinetic utilization of multiple daily tablet dosing with venlafaxine hydrochloride which comprises administering to a patient in need of treatment with venlafaxine hydrochloride, a one-a-day, extended release formulation of venlafaxine hydrochloride.

The use of the one-a-day venlafaxine hydrochloride formulations of this invention reduces by adaptation, the level of nausea and incidence of emesis that attend the administration of multiple daily dosing. In clinical trials of venlafaxine hydrochloride ER, the probability of developing nausea in the course of the trials was greatly reduced after the first week. Venlafaxine ER showed a statistically significant improvement over conventional venlafaxine hydrochloride tablets in two eight-week and one 12 week clinical studies. Thus, the invention is useful in there is provided a method for reducing the level of nausea and incidence of emesis attending the administration of venlafaxine hydrochloride which comprises dosing a patient in need of treatment with venlafaxine hydrochloride with an extended release formulation of venlafaxine hydrochloride once a day in a therapeutically effective amount.

Detailed Description of the Invention

1-[2-(dimethylamino)-1-(4-methoxyphenyl)ethyl]cyclohexanol hydrochloride, i.e. venlafaxine hydrochloride, is polymorphic. Of the forms isolated and characterized to date, Form I is considered to be the kinetic product of crystallization which can be converted to Form II upon heating in the crystallization solvent. Forms I and II cannot be distinguished by their melting points but do exhibit some differences in their infrared spectra and X-ray diffraction patterns. Any of the polymorphic forms such as Form I or Form II may be used in the formulations of the present invention. Microcrystalline cellulose, which is a component of the spheroid cores of the invention, may be any pharmaceutical grade of Microcrystalline Cellulose, NF, for example, Avicel® PH101.

In one aspect, this invention provides an improved core of the extended release spheroids comprised of venlafaxine hydrochloride and microcrystalline cellulose, which are free of hydroxypropylmethylcellulose. In one embodiment of this aspect, the spheroid core is comprised of from about 30 to about 40 percent by weight of venlafaxine hydrochloride, and preferably from about 35 to 40 percent by weight of venlafaxine hydrochloride. In a further embodiment of this aspect, the spheroid core is comprised of from about 6 to about 29 percent, preferably about 8 to about 25 percent, and more preferably from about 8 to about 18 percent, by weight of venlafaxine hydrochloride.

In another aspect of the invention, the spheroid core has a coating comprising ethyl cellulose and hydroxypropylmethylcellulose. The coating is preferably comprised of 80 to 90 percent of ethyl cellulose and 10 to 20 percent hydroxypropylmethylcellulose on a weight to weight basis. In the embodiment in which the spheroid core is comprised of from about 30 to about 40 percent by weight of venlafaxine hydrochloride, the coating is preferably of from about 2 to about 12 percent, and more preferably about 6 to about 8 percent, by weight of the total weight of the coated spheroid. In the embodiment in which the spheroid core is comprised of from about 6 to about 29 percent by weight of venlafaxine hydrochloride, the coating is preferably of from about 2 to about 8 percent, and more preferably about 3 to about 6 percent, by weight of the total weight of the coated spheroid.

In a further aspect, the invention provides an extended release formulation comprising a therapeutically effective amount of such coated spheroids containing venlafaxine hydrochloride and microcrystalline cellulose. Further, the invention provides encapsulated, extended release formulations comprising a therapeutically effective amount of such coated spheroids having the following dissolution profile in USP Apparatus 1 (basket) at 100 rpm in purified water at 37°C:

Table A

| Time (hours) | Average % Venlafaxine HCl released |
|---|---|
| 2 | <30 |
| 4 | 30-55 |
| 8 | 55-80 |
| 12 | 65-90 |
| 24 | >80. |

In a preferred embodiment there is provided a therapeutic spheroid comprising a core comprised of from about 30 to about 40 percent venlafaxine hydrochloride and from about 60 to about 70 percent microcrystalline cellulose and a coating comprised of from about 2 to about 12 percent of the total of a mixture of ethyl cellulose and hydroxypropylmethylcellulose, all on a weight to weight basis, said core preferably being substantially free of hydroxypropylmethylcellulose. In this embodiment, preferably, the core is comprised of from about 35 to about 40 percent venlafaxine hydrochloride and from about 60 to about 65 percent microcrystalline cellulose and the coating is comprised of from about 6 to about 8 percent of the total weight. In this preferred embodiment, the invention also provides an extended release formulation comprising a therapeutically effective amount of such coated spheroids and an encapsulated, extended release formulation comprising a therapeutically effective amount of such coated spheroids having the dissolution profile set out in Table A.

In a further preferred embodiment there is provided a therapeutic spheroid comprising a core comprised of from about 6 to about 29 percent venlafaxine hydrochloride and from about 94 to about 71 percent microcrystalline cellulose and a coating comprised of from about 2 to about 8 percent of the total of a mixture of ethyl cellulose and hydroxypropylmethylcellulose, all on a weight to weight basis, said core being free of hydroxypropylmethylcellulose. In this embodiment, preferably, the core is comprised of from about 8 to about 25 percent, and more preferably, from about 8 to about 18 percent, venlafaxine hydrochloride and from about 82 to about 75 percent, and more preferably about 82 to about 92 percent, microcrystalline cellulose, and the coating is preferably from about 6 to about 8 percent, and more preferably about 3 to about 6 percent, by weight of the coated spheroid total. In this preferred embodiment, the invention also provides an extended release formulation comprising a therapeutically effective amount of such coated spheroids. and an encapsulated, extended release formulation comprising a therapeutically effective amount of such coated spheroids having the dissolution profile set out in Table A.

In a further aspect, this invention provides a method of preparing the above-described spheroid cores, said method comprising extruding a mixture of venlafaxine hydrochloride and microcrystalline cellulose and then spheronising the resulting mixture. In a further step, such spheroid cores are coated with a mixture of ethyl cellulose and hydroxypropylmethylcellulose to provide the coated, extended release spheroids of the invention, preferably having the desired release rate characteristics. In a further step, the coated spheroids are sieved and selected spheroids are used to fill capsules to provide the extended release capsules of the invention.

The film coating is comprised of 80 to 90 percent of ethyl cellulose, NF and 10 to 20 percent hydroxypropyl methylcellulose, USP on a weight/weight basis. Preferably, the ethyl cellulose has a ethoxy content of 44.0-51% and a viscosity of 50 cps for a 5% aqueous solution, and preferably, the hydroxypropylmethylcellulose is USP 2910 having a viscosity of 6 cps at 2% aqueous solution with a methoxy content of 28-30% and a hydroxypropoxy content of 7-12%. The preferred ethyl cellulose used herein is Aqualon HG 2834. Other equivalents of the hydroxypropylmethylcellulose 2910 USP and ethyl cellulose, NF, having the same chemical and physical characteristics as the proprietary products named above may be substituted in the formulation without changing the inventive concept. (EP 0797991 A1 provided that hydroxypropylmethylcellulose 2208 USP, K3, Dow, which has a viscosity of 3 cps for 2% aqueous solutions, a methoxy content of 19-24% and a hydroxypropoxy content of 4-13%, was preferred for use in the spheroid core in order to make extrusion of the spheroids practical.)

This invention further provides a process for the manufacture of spheroid cores containing venlafaxine hydrochloride, which process comprises forming the spheroid cores from a composition that comprises venlafaxine hydrochloride and microcrystalline cellulose, the composition being free of hydroxypropylmethylcellulose. Such process also includes a further step comprising forming spheroids by coating the spheroid cores with a mixture of ethyl cellulose and hydroxypropylmethylcellulose. An additional comprises sieving the spheroids and using selected spheroids to fill capsules. Preferably, the foregoing process steps are ones in which the spheroid cores are formed by extruding a mixture of venlafaxine hydrochloride and microcrystalline cellulose and spheronizing the resulting mixture.

The following examples illustrate the practice of the invention without limiting its scope.

VENLAFAXINE HYDROCHLORIDE EXTENDED RELEASE CAPSULES WITH HPMC (Reference examples)

Example 1

A mixture of 44.8 parts ( 88.4 % free base) of venlafaxine hydrochloride, 74.6 parts of the microcrystalline cellulose, NF, and 0.60 parts of hydroxypropylmethyl cellulose 2208, USP, are blended with the addition of 41.0 parts water. The plastic mass of material is extruded, spheronized and dried to provide uncoated drug containing spheroids.

Stir 38.25 parts of ethyl cellulose, NF, HG2834 and 6.75 parts of hydroxypropyl methylcellulose 2910, USP in a 1:1 v/v mixture of methylene chloride and anhydrous methanol until solution of the film coating material is complete.

To a fluidized bed of the uncoated spheroids is applied 0.667 parts of coating solution per part of uncoated spheroids to obtain extended release, film coated spheroids having a coating level of 3%.

The spheroids are sieved to retain the coated spheroids of a particle size between 0.85 mm to 1.76 mm diameter. These selected film coated spheroids are filled into hard gelatin capsules conventionally.

Example 2

Same as for Example 1 except that 1.11 parts of the film coating solution per part of uncoated spheroids is applied to obtain a coating level of 5%.

Example 3

Same as for Example 1 except that 1.33 parts of the film coating solution is applied to 1 part of uncoated spheroids to obtain a coating level of 6%.

Example 4

Same as for Example 1 except that 1.55 parts of the film coating solution is applied to 1 part of uncoated spheroids to obtain a coating level of 7%.

The art-skilled person will appreciate that the composition can be practiced utilizing several types of conventional extruders. However, extruders having screw-type gravity feed and cylindrical gear discharge units (for example, Hutt Pellitizer model no. GCS-200/80) or gravity feed and a radial screen discharge unit (for example, Nica Extruder model no. E140) are preferred over those having a gravity feed and a cylindrical discharge unit (for example, Alexanderwerk model no. C1/100/1605).

VENLAFAXINE HYDROCHLORIDE EXTENDED RELEASE CAPSULES WITHOUT HPMC

Example 5

Spheroids of the invention were produced having 8.25% (w/w) venlafaxine hydrochloride and the remainder (91.75%, w/w) being microcrystalline cellulose, with a coating of from 3 to 5 % (w/w), preferably 4%, of the total weight. The spheroids with 8.25% venlafaxine hydrochloride and 4% coating were filled into No. 2 white opaque shells with a target fill weight of 236 mg.

Example 6

Further spheroids of the invention were produced having 16.5% (w/w) venlafaxine hydrochloride and the remainder (83.5%,w/w) being microcrystalline cellulose, with a coating of from 4 to 6 % (w/w), preferably 5%, of the total weight. The spheroids 16.5% venlafaxine hydrochloride and 5% coating were filled into No. 2 white opaque shells with a target fill weight of 122 mg.

The stability of capsules containing the 16.5% (w/w) strength of venlafaxine hydrochloride (in a 18.75 mg dosage capsule) without HPMC in the spheroid core is equivalent to that of such capsules of higher strengths of venlafaxine hydrochloride with HPMC in the spheroid core for up to six months. In both cases, at the end of six months, the venlafaxine strength remained at 98 percent or above of the original strength and there was less than one percent of detectable impurities. Further, with respect to capsules containing the 16.5% (w/w) strength of venlafaxine hydrochloride without HPMC in the spheroid core, efficacy was demonstrated, on a dose-related basis, in a human clinical trial.

The test for acceptability of the coating level is determined by analysis of the dissolution rate of the finished coated spheroids prior the encapsulation. The dissolution procedure followed uses USP Apparatus 1 (basket) at 100 rpm in purified water at 37°C.

Conformance with the dissolution rate given in Table 1 provides the twenty-four hour therapeutic blood levels for the drug component of the extended release capsules of this invention in capsule form. Where a given batch of coated spheroids releases drug too slowly to comply with the desired dissolution rate study, a portion of uncoated spheroids or spheroids with a lower coating level may be added to the batch to provide, after thorough mixing, a loading dose for rapid increase of blood drug levels. A batch of coated spheroids that releases the drug too rapidly can receive additional film-coating to give the desired dissolution profile.

Table I

| Acceptable Coated Spheroid Dissolution Rates | |
| --- | --- |
| Time (hours) | Average % Venlafaxine HCl released |
| 2 | <30 |
| 4 | 30-55 |
| 8 | 55-80 |
| 12 | 65-90 |
| 24 | >80 |

Batches of the coated venlafaxine hydrochloride containing spheroids which have a dissolution rate corresponding to that of Table 1 are filled into hard gelatin capsules in an amount needed to provide the unit dosage level desired. The standard unit dosage immediate release (IR) tablet used presently provides amounts of venlafaxine hydrochloride equivalent to 25 mg, 37.5 mg, 50 mg, 75 mg and 100 mg venlafaxine. The capsules of this invention are filled to provide an amount of venlafaxine hydrochloride equivalent to that presently used in tablet form and also up to about 150 mg venlafaxine hydrochloride.

Dissolution of the venlafaxine hydrochloride ER capsules is determined as directed in the U. S. Pharmacopoeia (USP) using apparatus 1 at 100 rpm on 0.9 L of water. A filtered sample of the dissolution medium is taken at the times specified. The absorbance of the clcar solution is determined from 240 to 450 nanometers (nm) against the dissolution medium. A baseline is drawn from 450 nm through 400 nm and extended to 240 nm. The absorbance at the wavelength of maximum absorbance (about 274 nm) is determined with respect to this baseline. Six hard gelatin capsules are filled with the theoretical amount of venlafaxine hydrochloride spheroids and measured for dissolution. Standard samples consist of venlafaxine hydrochloride standard solutions plus a gelatin capsule correction solution.

The percentage of venlafaxine released is determined from the equation

$$\% \text{ Venlafaxine hydrochloride released} = \frac{(As)(Wr)(S)(V1)(0.888)(100)}{(Ar)(V2)(C)}$$

where As is absorbance of sample preparation, Wr is weight of reference standard, mg; S is strength of the reference standard, decimal; V1 is the volume of dissolution medium used to dissolve the dosage form, ml; 0.884 is the percent free base, Ar is the absorbance of the standard preparation, V2 is the volume of reference standard solution, mL; and C is the capsule claim in mg.

Table 2 below is incorporated from EP 0797991 , which is a reference example, A1 and shows the plasma level of venlafaxine versus time for one 75 mg conventional Immediate Release (IR) tablet administered every 12 hours, two 75 mg extended release (ER) capsules administered simultaneously every 24 hours, and one 150 mg extended release (ER) capsule administered once every 24 hours in human male subjects. The subjects were already receiving venlafaxine hydrochloride according to the dosage protocol, thus the plasma blood level at zero time when dosages were administered is not zero. The ER capsules are those described in EP 0797991 A1 which have HPMC in the spheroids.

Table 2

| Plasma venlafaxine level (ng/mL) versus time, conventional tablet (not extended release) versus ER capsule | | | |
| --- | --- | --- | --- |
| Time (hours) | 75 mg (IR)tablet (q 12 h) | 2 x 75 mg (ER)capsules (q 24 hr) | 1 x 150 mg (ER)capsules (q 24 h) |
| 0 | 62.3 | 55.0 | 55.8 |
| 0.5 | 76.3 | | |

Table 2   (continued)

| Plasma venlafaxine level (ng/mL) versus time, conventional tablet (not extended release) versus ER capsule | | | |
|---|---|---|---|
| Time (hours) | 75 mg (IR)tablet (q 12 h) | 2 x 75 mg (ER)capsules (q 24 hr) | 1 x 150 mg (ER)capsules (q 24 h) |
| 1 | 135.6 | 53.3 | 53.2 |
| 2 | 212.1 | 69.8 | 70.9 |
| 4 | 162.0 | 138.6 | 133.3 |
| 6 | 114.6 | 149.0 | 143.5 |
| 8 | 86.7 | 129.3 | 129.5 |
| 10 | | 118.4 | 114.4 |
| 12 | 51.9 | 105.1 | 105.8 |
| 12.5 | 74.7 | | |
| 13 | 127.5 | | |
| 14 | 161.3 | 90.5 | 91.3 |
| 16 | 134.6 | 78.2 | 78.5 |
| 18 | 106.2 | | |
| 20 | 83.6 | 62.7 | 63.3 |
| 24 | 57.6 | 56.0 | 57.3 |

Table 2 shows that the plasma levels of two 75 mg/capsule venlafaxine hydrochloride ER capsules and one 150 mg/capsule venlafaxine hydrochloride ER capsule provide very similar blood levels. The data also show that the plasma level after 24 hours for either extended release regimen is very similar to that provided by two immediate release 75 mg tablets of venlafaxine hydrochloride administered at 12 hour intervals.

Further, the plasma levels of venlafaxine obtained with the extended release formulation do not increase to the peak levels obtained with the conventional immediate release tablets given 12 hours apart. The peak level of venlafaxine from (ER), somewhat below 150 ng/ml, is reached in about six hours, plus or minus two hours, based upon this specific dose when administered to patients presently under treatment with venlafaxine hydrochloride (IR). The peak plasma level of venlafaxine, somewhat over 200 ng/ml, following administration of (IR) is reached in two hours and falls rapidly thereafter.

Table 3 below is incorporated from EP 0797991 A1 ,which is a reference example, and shows venlafaxine blood plasma levels in male human subjects having a zero initial blood plasma level. Again, a peak blood plasma concentration of venlafaxine is seen at about 6 hours after dosing with venlafaxine hydrochloride extended release capsules in the quantities indicated. The subjects receiving the single 50 mg immediate release tablet showed a peak plasma level occurring at about 4 hours. For comparative purposes, the plasma levels of venlafaxine for subjects receiving the conventional formulated tablet can be multiplied by a factor of three to approximate the plasma levels expected for a single dose of 150 mg. conventional formulation. The ER capsules are those described in EP 0797991 A1 which have HPMC in the spheroids.

Table 3.

| Plasma Blood Levels in Human Males Having No Prior Venlafaxine Blood Level | | | |
|---|---|---|---|
| Time (Hours) | 1 x 50 mg IR tablet | 2 x 75 mg ER capsules | 1 x 150 mg ER capsule |
| 0 | 0 | 0 | 0 |
| 1 | 27.87 | 1.3 | 0 |
| 1.5 | 44.12 | 6.0 | 2.2 |
| 2 | 54.83 | 20.6 | 12.8 |
| 4 | 66.38 | 77.0 | 81.0 |
| 6 | 49.36 | 96.5 | 94.4 |
| 8 | 30.06 | 93.3 | 86.9 |
| 10 | 21.84 | 73.2 | 72.8 |
| 12 | 15.91 | 61.3 | 61.4 |
| 14 | 13.73 | 52.9 | 51.9 |
| 16 | 10.67 | 47.5 | 41.1 |

Table 3.   (continued)

| Plasma Blood Levels in Human Males Having No Prior Venlafaxine Blood Level | | | |
|---|---|---|---|
| Time (Hours) | 1 x 50 mg IR tablet | 2 x 75 mg ER capsules | 1 x 150 mg ER capsule |
| 20 | 5.52 | 35.2 | 34.0 |
| 24 | 3.56 | 29.3 | 28.5 |
| 28 | 2.53 | 23.4 | 22.9 |
| 36 | 1.44 | 11.9 | 13.5 |
| 48 | 0.66 | 5.8 | 5.2 |

The blood plasma levels of venlafaxine were measured according to the following procedure. Blood samples from the subjects were collected in heparinized evacuated blood tubes and the tubes were inverted gently several times. As quickly as possible, the tubes were centrifuged at 2500 rpm for 15 minutes. The plasma was pipetted into plastic tubes and stored at -20°C until analysis could be completed.

To 1 mL of each plasma sample in a plastic tube was added 150 μL of a stock internal standard solution (150 μg/ml). Saturated sodium borate (0.2 mL) solution was added to each tube and vortexed. Five mL of ethyl ether was added to each tube which were then capped and shaken for 10 minutes at high speed. The tubes were centrifuged at 3000 rpm for 5 minutes. The aqueous layer was frozen in dry ice and the organic layer transferred to a clean screw cap tube. A 0.3 mL portion of 0.01 N HCl solution was added to each tube and shaken for 10 minutes at high speed. The aqueous layer was frozen and the organic layer removed and discarded. A 50μL portion of the mobile phase (23: 77 acetonitrile:0.1M monobasic ammonium phosphate buffer, pH 4.4) was added to each tube, vortexed, and 50 μL samples were injected on a Supelco Supelcoil LC-8-DB, 5 cm x 4.6 mm, 5 μ column in a high pressure liquid chromatography apparatus equipped with a Waters Lambda Max 481 detector or equivalent at 229 nm. Solutions of venlafaxine hydrochloride at various concentrations were used as standards.

Thus, the desired dissolution rate of a sustained release dosage form of venlafaxine hydrochloride, impossible to achieve with hydrogel tablet technology, has been achieved with the further film-coated spheroid compositions.

**Claims**

1.   A spheroid core comprising venlafaxine hydrochloride and microcrystalline cellulose, in which the spheroid core does not contain hydroxypropylmethylcellulose.

2.   A spheroid core as claimed in claim 1 comprised of from 30 to 40 percent by weight of venlafaxine hydrochloride.

3.   A spheroid core as claimed in claim 1 comprised of from 6 to 29 percent by weight of venlafaxine hydrochloride.

4.   A spheroid core as claimed in claim 1 comprised of from 8 to 25 percent by weight of venlafaxine hydrochloride.

5.   A spheroid core as claimed in claim 1 comprised of from 8 to 18 percent by weight of venlafaxine hydrochloride.

6.   A spheroid comprising a spheroid core as claimed in any one of claims 1 to 5 which has a coating comprising ethyl cellulose and hydroxypropylmethylcellulose.

7.   A spheroid as claimed in claim 6 wherein the coating is comprised of 80 to 90 percent of ethyl cellulose and 10 to 20 percent hydroxypropyl methylcellulose on a weight to weight basis.

8.   A spheroid as claimed in claim 6 comprising a spheroid core as claimed in claim 2 with a coating of from 2 to 12 percent, by weight of the total, of a mixture of ethyl cellulose and hydroxypropylmethylcellulose.

9.   A spheroid as claimed in claim 6 comprising a spheroid core as claimed in claim 2 with a coating of from 6 to 8 percent, by weight of the total, of a mixture of ethyl cellulose and hydroxypropylmethylcellulose.

10.   A spheroid as claimed in claim 6 comprising a spheroid core as claimed in any one of claims 3 to 5 with a coating of from 2 to 8 percent, by weight of the total, of a mixture of ethyl cellulose and hydroxypropylmethylcellulose.

11. A spheroid as claimed in claim 6 comprising a spheroid core as claimed in any one of claims 3 to 5 with a coating of from 3 to 6 percent, by weight of the total, of a mixture of ethyl cellulose and hydroxypropylmethylcellulose.

12. A therapeutic spheroid as claimed in claim 6 comprising a core comprised of from 30 to 40 percent venlafaxine hydrochloride and from 60 to 70 percent microcrystalline cellulose and a coating comprised of from 2 to 12 percent of the total of a mixture of ethyl cellulose and hydroxypropylmethylcellulose, all on a weight to weight basis.

13. A therapeutic spheroid as claimed in claim 12, wherein the core is comprised of from 35 to 40 percent venlafaxine hydrochloride and from 60 to 65 percent microcrystalline cellulose and the coating is comprised of from 6 to 8 percent of the total of a mixture of ethyl cellulose and hydroxypropylmethylcellulose, all on a weight to weight basis.

14. A therapeutic spheroid as claimed in claim 6 comprising a core comprised of from 6 to 29 percent venlafaxine hydrochloride and from 94 to 71 percent microcrystalline cellulose and a coating comprised of from 2 to 8 percent of the total of a mixture of ethyl cellulose and hydroxypropylmethylcellulose, all on a weight to weight basis.

15. A therapeutic spheroid as claimed in claim 14, wherein the core is comprised of from 8 to 25 percent venlafaxine hydrochloride and from 92 to 75 percent microcrystalline cellulose and the coating is comprised of from 3 to 6 percent of a mixture of ethyl cellulose and hydroxypropylmethylcellulose, all on a weight to weight basis

16. An extended release formulation comprising a therapeutically effective amount of coated spheroids as claimed in any one of claims 6-11.

17. An extended release formulation as claimed in claim 16 which is encapsulated and has the following dissolution profile in USP Apparatus 1 (basket) at 100 rpm in purified water at 37°C:

| Time (hours) | Average % Venlafaxine HCl released |
|---|---|
| 2 | <30 |
| 4 | 30-55 |
| 8 | 55-80 |
| 12 | 65-90 |
| 24 | >80. |

18. An extended release formulation as claimed in claim 16 comprising a therapeutically effective amount coated spheroids as claimed in claim 12 or 13.

19. An extended release formulation as claimed in claim 18 which is encapsulated and has the following dissolution profile in USP Apparatus 1 (basket) at 100 rpm in purified water at 37°C:

| Time (hours) | Average % Venlafaxine HCl released |
|---|---|
| 2 | <30 |
| 4 | 30-55 |
| 8 | 55-80 |
| 12 | 65-90 |
| 24 | >80. |

20. An extended release formulation comprising a therapeutically effective amount of coated spheroids as claimed in claim 14 or 15.

21. An extended release formulation as claimed in claim 20 which is encapsulated and has the following dissolution profile in USP Apparatus 1 (basket) at 100 rpm in purified water at 37°C:

| Time (hours) | Average % Venlafaxine HCl released |
|---|---|
| 2 | <30 |
| 4 | 30-55 |

(continued)

| Time (hours) | Average % Venlafaxine HCl released |
|---|---|
| 8 | 55-80 |
| 12 | 65-90 |
| 24 | >80. |

22. A method of preparing a spheroid core as claimed in any one of claims 1 to 5 by extruding a mixture of venlafaxine hydrochloride and microcrystalline cellulose and then spheronising the resulting mixture.

23. A method of preparing a spheroid as claimed in any one of claims 6 to 11 comprising the method as claimed in claim 22 and the further step of coating the spheroid cores with a mixture of ethyl cellulose and hydroxypropylmethylcellulose.

24. A method of preparing an extended release composition as claimed in Claim 18 which is encapsulated, comprising a method as claimed in claim 23 and the further step of sieving the spheroids and using selected spheroids to fill capsules.

**Patentansprüche**

1. Sphäroidkern, welcher Venlafaxin-hydrochlorid und mikrokristalline Cellulose umfasst, wobei der Sphäroidkern keine Hydroxypropylmethylcellulose enthält.

2. Sphäroidkern wie in Anspruch 1 beansprucht, welcher 30 bis 40 Gewichtsprozent Venlafaxin-hydrochlorid umfasst.

3. Sphäroidkern wie in Anspruch 1 beansprucht, welcher von 6 bis 29 Gewichtsprozent Venlafaxin-hydrochlorid umfasst.

4. Sphäroidkern wie in Anspruch 1 beansprucht, welcher von 8 bis 25 Gewichtsprozent Venlafaxin-hydrochlorid umfasst.

5. Sphäroidkern wie in Anspruch 1 beansprucht, welcher von 8 bis 18 Gewichtsprozent Venlafaxin-hydrochlorid umfasst.

6. Sphäroid, welches einen Sphäroidkern umfasst, wie in einem der Ansprüche 1 bis 5 beansprucht, welches einen Überzug hat, welcher Ethylcellulose und Hydroxypropylmethylcellulose umfasst.

7. Sphäroid wie in Anspruch 6 beansprucht, wobei der Überzug 80 bis 90 Prozent Ethylcellulose und 10 bis 20 Prozent Hydroxypropylmethylcellulose auf einer Gewicht-zu-Gewicht Basis umfasst.

8. Sphäroid wie in Anspruch 6 beansprucht, welches einen Sphäroidkern wie in Anspruch 2 beansprucht umfasst, mit einem Überzug aus von 2 bis 12 Gesamtgewichtsprozent aus einem Gemisch aus Ethylcellulose und Hydroxypropylmethylcellulose.

9. Sphäroid wie in Anspruch 6 beansprucht, welches einen Sphäroidkern wie in Anspruch 2 beansprucht umfasst, mit einem Überzug aus von 6 bis 8 Gesamtgewichtsprozent aus einem Gemisch aus Ethylcellulose und Hydroxypropylmethylcellulose.

10. Sphäroid wie in Anspruch 6 beansprucht, welches einen Sphäroidkern wie in einem der Ansprüche 3 bis 5 beansprucht umfasst, mit einem Überzug aus von 2 bis 8 Gesamtgewichtsprozent aus einem Gemisch aus Ethylcellulose und Hydroxypropylmethylcellulose.

11. Sphäroid wie in Anspruch 6 beansprucht, welches einen Sphäroidkern wie in einem der Ansprüche 3 bis 5 beansprucht umfasst, mit einem Überzug aus von 3 bis 6 Gesamtgewichtsprozent aus einem Gemisch aus Ethylcellulose und Hydroxypropylmethylcellulose.

12. Therapeutisches Sphäroid wie in Anspruch 6 beansprucht, welches einen Kern, welcher 30 bis 40 Prozent Venlafaxin-hydrochlorid und von 60 bis 70 Prozent mikrokristalline Cellulose und einen Überzug umfasst, welcher 2 bis 12 Gesamtgewichtsprozent eines Gemisches aus Ethylcellulose und Hydroxypropylmethylcellulo se umfasst, alles auf einer Gewicht-zu-Gewicht Basis.

13. Therapeutisches Sphäroid wie in Anspruch 12 beansprucht, wobei der Kern 35 bis 40 Prozent Venlafaxin-hydrochlorid und von 60 bis 65 Prozent mikrokristalline Cellulose umfasst und der Überzug von 6 bis 8 Gesamtgewichtsprozent eines Gemisches aus Ethylcellulose und Hydroxypropylmethylcellulose umfasst, alles auf einer Gewicht-zu-Gewicht Basis.

14. Therapeutisches Sphäroid wie in Anspruch 6 beansprucht, umfassend einen Kern, welcher von 6 bis 29 Prozent Venlafaxin-hydrochlorid und von 94 bis 71 Prozent mikrokristalline Cellulose umfasst und einen Überzug, welcher von 2 bis 8 Gesamtgewichtsprozent eines Gemisches aus Ethylcellulose und Hydroxypropyl-methylcellulose umfasst, alles auf einer Gewicht-zu-Gewicht Basis.

15. Therapeutisches Sphäroid wie in Anspruch 14, wobei der Kern von 8 bis 25 Prozent Venlafaxin-hydrochlorid und von 92 bis 75 Prozent mikrokristalliner Cellulose umfasst und der Überzug von 3 bis 6 Prozent eines Gemisches aus Ethylcellulose und Hydroxypropylmethylcellulose umfasst, alles auf einer Gewicht-zu-Gewicht Basis.

16. Formulierung mit verlängerter Abgabe, welche eine therapeutisch wirksame Menge überzogener Sphäroide wie in einem der Ansprüche 6-11 beansprucht umfasst.

17. Formulierung mit verlängerter Abgabe wie in Anspruch 16 beansprucht, welche eingekapselt ist und das folgende Auflösungsprofil im USP-Apparatus 1 (Korb) bei 100 U/min in gereinigtem Wasser bei 37°C hat:

| Zeit (Stunden) | Durchschnittliche % an abgege-benem Venlafaxin-HCl |
|---|---|
| 2 | <30 |
| 4 | 30-55 |
| 8 | 55-80 |
| 12 | 65-90 |
| 24 | >80 |

18. Formulierung mit verlängerter Abgabe wie in Anspruch 16 beansprucht, welche eine therapeutisch wirksame Menge überzogener Sphäroide wie in Anspruch 12 oder 13 beansprucht umfasst.

19. Formulierung mit verlängerter Abgabe wie in Anspruch 18 beansprucht, welche eingekapselt ist und das folgende Auflösungsprofil im USP-Apparatus 1 (Korb) bei 100 U/min in gereinigtem Wasser bei 37°C hat:

| Zeit (Stunden) | Durchschnittliche % an abgege-benem Venlafaxin-HCl |
|---|---|
| 2 | <30 |
| 4 | 30-55 |
| 8 | 55-80 |
| 12 | 65-90 |
| 24 | >80 |

20. Formulierung mit verlängerter Abgabe, welche eine therapeutisch wirksame Menge überzogener Sphäroide wie in Anspruch 14 oder 15 beansprucht umfasst.

21. Formulierung mit verlängerter Abgabe wie in Anspruch 20 beansprucht, welche eingekapselt ist und das folgende Auflösungsprofil im USP-Apparatus 1 (Korb) bei 100 U/min in gereinigtem Wasser bei 37°C hat:

| Zeit (Stunden) | Durchschnittliche % an abgege-benem Venlafaxin-HCl |
|---|---|
| 2 | <30 |
| 4 | 30-55 |

(fortgesetzt)

| Zeit (Stunden) | Durchschnittliche % an abgege-benem Venlafaxin-HCl |
|---|---|
| 8 | 55-80 |
| 12 | 65-90 |
| 24 | >80 |

**22.** Verfahren zum Herstellen eines Sphäroidkerns wie in einem der Ansprüche 1 bis 5 beansprucht, durch Extrudieren eines Gemisches aus Venlafaxin-hydrochlorid und mikrokristalliner Cellulose und dann Sphäronisieren des sich ergebenden Gemisches.

**23.** Verfahren zum Herstellen eines Sphäroids wie in einem der Ansprüche 6 bis 11 beansprucht, welches das Verfahren wie in Anspruch 22 beansprucht und den weiteren Schritt, Überziehen des Sphäroidkerns mit einem Gemisch aus Ethylcellulose und Hydroxypropylmethylcellulose, umfasst.

**24.** Verfahren zum Herstellen einer Zusammensetzung mit verlängerter Abgabe wie in Anspruch 18 beansprucht, welche eingekapselt ist, welches ein Verfahren wie in Anspruch 23 beansprucht und den weiteren Schritt, Sieben der Sphäroide und Verwenden ausgewählter Sphäroide, um Kapseln zu füllen, umfasst.

**Revendications**

**1.** Noyau sphéroïde comprenant du chlorhydrate de venlafaxine et de la cellulose microcristalline, dans lequel le noyau sphéroïde ne contient pas d'hydroxypropylméthylcellulose.

**2.** Noyau sphéroïde suivant la revendication 1, comprenant de 30 à 40 pour-cent en poids de chlorhydrate de venlafaxine.

**3.** Noyau sphéroïde suivant la revendication 1, comprenant de 6 à 29 pour-cent en poids de chlorhydrate de venlafaxine.

**4.** Noyau sphéroïde suivant la revendication 1, comprenant de 8 à 25 pour-cent en poids de chlorhydrate de venlafaxine.

**5.** Noyau sphéroïde suivant la revendication 1, comprenant de 8 à 18 pour-cent en poids de chlorhydrate de venlafaxine.

**6.** Corps sphéroïde comprenant un noyau sphéroïde suivant l'une quelconque des revendications 1 à 5, qui présente un enrobage comprenant de l'éthylcellulose et de l'hydroxypropylméthylcellulose.

**7.** Corps sphéroïde suivant la revendication 6, dans lequel l'enrobage est constitué de 80 à 90 pour-cent d'éthylcellulose et de 10 à 20 pour-cent d'hydroxypropylméthylcellulose sur une base de poids sur poids.

**8.** Corps sphéroïde suivant la revendication 6, comprenant un noyau sphéroïde suivant la revendication 2, avec un enrobage de 2 à 12 pour-cent, en poids au total, d'un mélange d'éthylcellulose et d'hydroxypropylméthylcellulose.

**9.** Corps sphéroïde suivant la revendication 6, comprenant un noyau sphéroïde suivant la revendication 2, avec un enrobage de 6 à 8 pour-cent, en poids au total, d'un mélange d'éthylcellulose et d'hydroxypropylméthylcellulose.

**10.** Corps sphéroïde suivant la revendication 6, comprenant un noyau sphéroïde suivant l'une quelconque des revendications 3 à 5, avec un enrobage de 2 à 8 pour-cent, en poids au total, d'un mélange d'éthylcellulose et d'hydroxypropylméthylcellulose.

**11.** Corps sphéroïde suivant la revendication 6, comprenant un noyau sphéroïde suivant l'une quelconque des revendications 3 à 5, avec un enrobage de 3 à 6 pour-cent, en poids au total, d'un mélange d'éthylcellulose et d'hydroxypropylméthylcellulose.

**12.** Corps sphéroïde thérapeutique suivant la revendication 6, comprenant un noyau constitué de 30 à 40 pour-cent de chlorhydrate de venlafaxine et de 60 à 70 pour-cent de cellulose microcristalline et un enrobage comprenant de 2 à 12 pour-cent au total d'un mélange d'éthylcellulose et d'hydroxypropylméthylcellulose, le tout sur une base de poids sur poids.

**13.** Corps sphéroïde thérapeutique suivant la revendication 12, dans lequel le noyau est constitué de 35 à 40 pour-cent de chlorhydrate de venlafaxine et de 60 à 65 pour-cent de cellulose microcristalline et l'enrobage comprend de 6 à 8 pour-cent au total d'un mélange d'éthylcellulose et d'hydroxypropylméthylcellulose, le tout sur une base de poids sur poids.

**14.** Corps sphéroïde thérapeutique suivant la revendication 6, comprenant un noyau constitué de 6 à 29 pour-cent de chlorhydrate de venlafaxine et de 94 à 71 pour-cent de cellulose microcristalline et un enrobage comprenant de 2 à 8 pour-cent au total d'un mélange d'éthylcellulose et d'hydroxypropylméthylcellulose, le tout sur une base de poids sur poids.

**15.** Corps sphéroïde thérapeutique suivant la revendication 14, dans lequel le noyau est constitué de 8 à 25 pour-cent de chlorhydrate de venlafaxine et de 92 à 75 pour-cent de cellulose microcristalline et l'enrobage comprend de 3 à 6 pour-cent d'un mélange d'éthylcellulose et d'hydroxypropylméthylcellulose, le tout sur une base de poids sur poids.

**16.** Formulation à libération prolongée comprenant une quantité efficace sur le plan thérapeutique de corps sphéroïdes enrobés suivant l'une quelconque des revendications 6-11.

**17.** Formulation à libération prolongée suivant la revendication 16, qui est encapsulée et présente le profil suivant de dissolution dans un appareillage USP 1 (à paniers) à 100 tr/min dans de l'eau purifiée à 37°C :

| Temps (heures) | % moyen libéré de venlafaxine HCl |
|---|---|
| 2 | <30 |
| 4 | 30-55 |
| 8 | 55-80 |
| 12 | 65-90 |
| 24 | >80 |

**18.** Formulation à libération prolongée suivant la revendication 16, comprenant une quantité efficace sur le plan thérapeutique de corps sphéroïdes enrobés suivant la revendication 12 ou 13.

**19.** Formulation à libération prolongée suivant la revendication 18, qui est encapsulée et présente le profil suivant de dissolution dans un appareillage USP 1 (à paniers) à 100 tr/min dans de l'eau purifiée à 37°C :

| Temps (heures) | % moyen libéré de venlafaxine HCl |
|---|---|
| 2 | <30 |
| 4 | 30-55 |
| 8 | 55-80 |
| 12 | 65-90 |
| 24 | >80 |

**20.** Formulation à libération prolongée comprenant une quantité efficace sur le plan thérapeutique de corps sphéroïdes enrobés suivant la revendication 14 ou 15.

**21.** Formulation à libération prolongée suivant la revendication 20, qui est encapsulée et présente le profil suivant de dissolution dans un appareillage USP 1 (à paniers) à 100 tr/min dans de l'eau purifiée à 37°C :

| Temps (heures) | % moyen libéré de Venlafaxine HCl |
|---|---|
| 2 | <30 |

(suite)

| Temps (heures) | % moyen libéré de Venlafaxine HCl |
|---|---|
| 4 | 30-55 |
| 8 | 55-80 |
| 12 | 65-90 |
| 24 | >80 |

22. Procédé de préparation d'un noyau sphéroïde suivant l'une quelconque des revendications 1 à 5, par l'extrusion d'un mélange de chlorhydrate de venlafaxine et de cellulose microcristalline puis par l'obtention de corps sphéroïdes à partir du mélange obtenu.

23. Procédé de préparation d'un corps sphéroïde suivant l'une quelconque des revendications 6 à 11, comprenant le procédé suivant la revendication 22, et l'étape supplémentaire d'enrobage des noyaux sphéroïdes avec un mélange d'éthylcellulose et d'hydroxypropylméthylcellulose.

24. Procédé de préparation d'une composition à libération prolongée suivant la revendication 18, qui est encapsulée, comprenant le procédé suivant la revendication 23 et l'étape supplémentaire du tamisage des corps sphéroïdes et de l'utilisation des corps sphéroïdes sélectionnés pour remplir des capsules.